# EUROPEAN PATENT SPECIFICATION

(11) **EP 1 728 508 B1**
(45) Date of publication and mention of the grant of the patent: **09.09.2009**
(21) Application number: 05721012.2
(22) Date of filing: 17.03.2005
(51) Int. Cl.: A61K 31/137, A61K 31/18, A61K 31/505, A61K 45/00, A61P 13/00

(54) **MEDICINE FOR PREVENTION OR TREATMENT OF FREQUENT URINATION OR URINARY INCONTINENCE**
MEDIKAMENT ZUR PRÄVENTION ODER BEHANDLUNG VON HÄUFIGEM HARNDRANG ODER HARNINKONTINENZ
MÉDICAMENT POUR LA PRÉVENTION OU LE TRAITEMENT DE LA MICTION FRÉQENTE OU DE L'INCONTINENCE URINAIRE

(30) Priority: 24.03.2004 JP 2004004000
(43) Date of publication of application: 06.12.2006
(73) Proprietor: Kissei Pharmaceutical Co., Ltd., Matsumoto-shi Nagano 399-8710 (JP)
(72) Inventor: YAMAZAKI, Yoshinobu, c/o Central Res. Lab., Minamiazumi-gun, Nagano 3998304 (JP); KOJIMA, Masami, c/o Central Res. Lab., Minamiazumi-gun, Nagano 3998304 (JP)
(74) Representative: Hayes, Adrian Chetwynd
(86) International application number: PCT/JP2005/004825
(87) International publication number: WO 2005/089742

(56) References cited:
- EP-A- 1 724 257
- WO-A-20/05042021
- WO-A1-00/02846
- WO-A1-03/024916
- WO-A2-02/069906
- JP-A- 1 026 517
- JP-A- 2001 114 679
- JP-A- 2001 288 115
- JP-A- 2002 338 513
- JP-A- 2003 055 261

## Description

### Technical Field

The present invention relates to medicines useful for the prevention or treatment of urinary frequency or incontinence. More particularly, the present invention relates to medicines for use in the prevention or treatment of urinary frequency or incontinence, characterized by comprising combination of a phenoxyacetic acid derivative represented by a general formula: 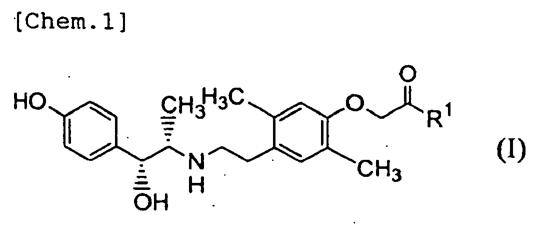 wherein R¹ represents a hydroxy group or a lower alkoxy group, or a pharmaceutically acceptable salt thereof or a hydrate or solvate thereof and an α₁-adrenoceptor (herein after sometimes referred to as α1-AR) blocker, namely silodosin.

### Background Art

In recent years, people who complain of lower urinary tract symptoms such as urinary frequency, incontinence or the like have been increasing according to concern about the QOL in micturition arising with progression of aging. Diseases producing lower urinary tract symptoms are wide-ranging, and many elderly people visit medical facilities mainly complaining their urinary frequency and incontinence. Now, in the treatment of the urinary frequency or incontinence, combination of behavioral modification to establish the normal voiding pattern such as timed voiding training, pelvic floor muscle training or education for patients and medication is commonly used. But anticholinergic drugs mainly used in medication have the possibility of side effects such as dry mouth, constipation, voiding dysfunction, central nervous system symptoms or the like, and thus the therapy is sometimes not able to continue or the therapeutic efficacy is sometimes insufficient (see non-Patent Reference 1).

On the other hand, a series of compounds containing the phenoxyacetic acid derivatives represented by the general formula (I) of the present invention which have an excellent β3-adrenoceptor (hereinafter sometimes referred to as β3-AR) stimulating activity have been developed, and novel drugs to prevent or treat the urinary frequency, incontinence or the like by exerting activities to relax the detrusor and increase the bladder capacity and urine storage volume have been proposed (see Patent Reference 1).

By the way, in benign prostatic hypertrophy (BPH), α1-ARs mainly distributing in smooth muscle of prostate and urethra increase. It is known that α1-AR blockers such as silodosin, tamsulosin, urapidil or the like improve the urethral resistance because the urethral smooth muscle is relaxed by their α1-AR blocking activities. For example, the usefulness for dysuria associated with BPH on silodosin (see Patent Reference 2), for dysuria associated with BPH (see Patent Reference 3), urinary dysfunction associated with functional obstruction of lower urinary tract (see Patent Reference 4) and voiding dysfunction associated with neurogenic bladder (see Patent Reference 5) on tamsulosin, and for urinary dysfunction associated with neurogenic bladder on urapidil (see Patent Reference 2) have been reported, respectively. However, there are neither any reports that α1-AR blockers have activities decreasing the intra-bladder pressure or prolonging the micturition interval nor any suggestions about a medicine comprising combination of an α1-AR blocker and a compound represented by the general formula (I) in these references. In addition, in Patent Reference 6, it is mentioned that various drugs including a β3-AR stimulant, an α1-AR blocker and the like can be used in combination for pain, inflammation or the like of urinary and sexual organs. But any combined use of a β3-AR stimulant and an α1-AR blocker is not specifically described, and it is not also described that such a use is effective for the prevention or treatment of the urinary frequency and/or incontinence in the reference.

In these situations, an effective drug for the prevention or treatment of the urinary frequency and/or incontinence has been increasingly desired, because it is expected that the numbers of not only total patients but also severe cases will increase with the increase in elderly people.
Patent Reference 1: International Publication WO00/02846 pamphlet;
Patent Reference 2: International Publication WO99/15202 pamphlet;
Patent Reference 3: Japanese Examined Patent Publication (Tokkosho) JP1987-52742 B;
Patent Reference 4: Japanese Patent publication (Tokkai) JP2001-288115 A;
Patent Reference 5: International Publication WO00/00187 pamphlet;
Patent Reference 6: International Publication WO02/069906 pamphlet;
Non-patent Reference 1: Scope, published by Pharmacia Company, 2003, Vol. 42, No.1, pp. 14-15;
Non-patent Reference 2: Iyaku Journal, published by Iyaku-Journal Company, 1997, Vol. 33 No.S-1, pp. 193-197.
WO2005/042021 describes a combination for the treatment of bladder disorders, which contains an alpha-antagonist and/or a 5-alpha reductase inhibitor and a beta-3-adrenoceptor agonist, where the alpha-antagonist is selected from tamsulosin, prazosin, terazosin and naftopidil.

### Disclosure of the Invention

### Problem to be Solved by the Invention

The object of the present invention is to provide medicines useful for the prevention or treatment of urinary frequency or incontinence.

### Means of solving the Problems

The present inventors have studied earnestly to resolve the above problems on a drug for the prevention or treatment of urinary frequency or incontinence, and found that an α1-AR blocker surprisingly shows an effect to decrease the intra-bladder pressure. Furthermore, a combination of a phenoxyacetic acid derivative represented by the general formula (I) and an α1-AR blocker enhances each other's efficacy decreasing the intra-bladder pressure or prolonging the micturition interval and exerts more remarkable effects in comparison with administration of either drug alone, thereby forming the basis of the present invention.

The present invention provides a medicine comprising combination of the later identified phenoxyacetic acid derivative or a pharmaceutically acceptable salt thereof, or a hydrate or solvate thereof and silodosin, which exerts an excellent effect decreasing the intra-bladder pressure or prolonging the micturition interval and is useful as an agent for the prevention of treatment or urinary frequency or incontinence.

That is, the present invention relates to:
[1] a medicine for use in the prevention or treatment of urinary frequency or incontinence, which comprises combination of a phenoxyacetic acid derivative represented by the general formula: 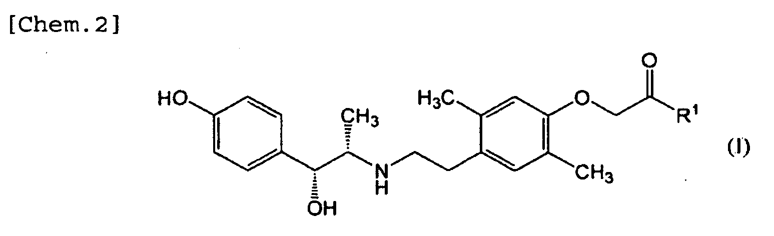 wherein R¹ represents a hydroxy group or a lower alkoxy group, or a pharmaceutically acceptable salt thereof, or a hydrate or solvate thereof and silodosin, wherein the dosage of silodosin or a pharmaceutically acceptable salt thereof is 1 to 16 mg/day as oral dose for an adult human.
[2] a medicine as described in the above [1] wherein the phenoxyacetic acid derivative represented by the general formula (I) is ethyl (-)-2-[4-[2-[[(1S, 2R)-2-hydroxy-2-(4-hydroxyphenyl)-1-methylethyl]amino]ethyl]-2,5-dimethyl-phenoxy]acetate;
[3] a combination formulation for the prevention or treatment of urinary frequency or incontinence, which comprises a phenoxyacetic acid derivative represented by the general formula (I) or a pharmaceutically acceptable salt thereof, or a hydrate or solvate thereof and silodosin, wherein the dosage of silodosin or a pharmaceutically acceptable salt thereof is 1 to 16 mg/day as oral dose for an adult human;
[4] a combination formulation as described in the above [3] wherein the phenoxyacetic acid derivative represented by the general formula (I) is ethyl (-)-2-[4-[2-[[(1S,2R)-2-hydroxy-2-(4-hydroxyphenyl)-1-methylethyl]amino]ethyl]-2,5-dimethylphenoxy]acetate.

More particularly, as mentioned below, a combined use of a phenoxyacetic acid derivative represented by the general formula (I) and silodosin exerted a more remarkable effect decreasing the intra-bladder pressure in comparison with administration of either drug alone in an intra-bladder pressure measurement in anesthetized rats, and furthermore, showed a more remarkable effect prolonging the micturition interval in comparison with administration of either drug alone in micturition interval measurement in acetic acid-stimulated rats. Therefore, the combined use of a phenoxyacetic acid derivative represented by the general formula (I) and silodosin is extremely effective for the prevention or treatment of urinary frequency or incontinence.

In the general formula (I), as a lower alkoxy group in R¹, for example, a straight-chained or branched alkoxyl group having 1 to 6 carbon atoms such as a methoxy group, an ethoxy group, a propoxy group, a butyloxy group, an isobutyloxy group, a sec-butyloxy group, a *tert*-butyloxy group, a pentyloxy group, an isopentyloxy group, a hexyloxy group and the like can be illustrated.

As the phenoxyacetic acid derivative represented by the general formula (I), ethyl (-)-2-[4-[2-[[(1S,2R)-2-hydroxy-2-(4-hydroxyphenyl)1-methylethyl]amino]ethyl]-2,5-dimethylphenoxy]acetate (hereinafter referred to as "compound 1") is preferable.

The phenoxyacetic acid derivatives represented by the general formula (I) can be prepared in a manner described in literatures or the like (for example, see Patent Reference 1)

The phenoxyacetic acid derivatives represented by the general formula (I) can be converted into pharmaceutically acceptable salts thereof in the usual way. As such a salt thereof, for example, a salt with an inorganic acid such as hydrochloric acid, hydrobromic acid, hydroiodic acid, sulfuric acid, nitric acid, phosphoric acid, carbonic acid or the like; a salt with a carboxylic acid such as formic acid, acetic acid, propionic acid, citric acid, succinic acid, tartaric acid, fumaric acid, butyric acid, oxalic acid, malonic acid, maleic acid, lactic acid, malic acid, glutamic acid, aspartic acid or the like; a salt with a sulfonic acid such as methanesulfonic acid, benzenesulfonic acid, p-toluenesulfonic acid or the like; a salt with an inorganic base such as a salt with an alkaline metal such as sodium, potassium or the like, a salt with an alkaline earth metal such as calcium or the like; and a salt with an organic base such as triethylamine, piperidine, morpholine, pyridine, lysine or the like; and the like can be illustrated.

The dosage of a phenoxyacetic acid derivative represented by the general formula (I) or a pharmaceutically acceptable salt thereof, or a hydrate or solvate thereof can be appropriately decided depending on the body weight, age, sex and degree of diseases of each patient and the α1-AR blocker, namely silodosin to be used in combination therewith, which is approximately within the range of from 1 to 1000 mg per day in the case of oral administration and approximately within the range of from 0.01 to 100 mg per day in the case of parenteral administration per adult human.

The dosage of silodosin can be appropriately decided depending on the body weight, age, sex and degree of diseases of each patient, which is approximately within the range of from 1 to 16 mg per day for silodosin, per adult human, in the case of oral administration.

A medicine comprising combination of a phenoxyacetic acid derivative represented by the general formula (I) and silodosin includes either dosage forms of a single preparation comprising both of the phenoxyacetic acid derivative and silodosin, and a combination formulation consisting of separated preparations of the phenoxyacetic acid derivative and silodosin for simultaneous administration or administration at different dosage intervals. In addition, when the combination formulation is used, both separated preparations can be administered in way of the same or different administration route.

The medicines comprising the phenoxyacetic acid derivative and silodosin can be prepared by admixing the phenoxyacetic acid derivative and silodosin with an appropriate pharmaceutical carrier such as excipients, disintegrators, binders, lubricants, diluents, buffers, isotonicities, antiseptics, moistening agents, emulsifiers, dispersing agents, stabilizing agents, dissolving aids and the like in various forms in accordance with conventional methods. In addition, each formulation of the phenoxyacetic acid derivative or silodosin available separately can be used for the combination formulation comprising the phenoxyacetic acid derivative and silodosin.

In addition, the combinatorial pharmaceutical composition of the present invention can be used in combination with another medicine useful for urinary frequency or incontinence as occasion demands. As the other medicine useful for urinary frequency or incontinence, for example, anticholinergics, β2-adrenoceptor agonists, estrogen preparations, drugs for central nervous system (selective serotonin reuptake inhibitors, serotonin-norepinephrine reuptake inhibitors or the like), neurokinin receptor antagonists, potassium channel openers, vanilloid receptor agonists, vasopressin 2 receptor agonists, GABA receptor agonists, serotonin receptor antagonists, dopamine receptor agonists, anti-allergic drugs, nonsteroidal anti-inflammatory drugs, NO synthesis inhibitors and the like can be illustrated.

The combined use of the phenoxyacetic acid derivative and silodosin remarkably decreases the intra-bladder pressure or remarkably prolongs the micturition interval, and therefore, exerts extremely high efficacy for the prevention or treatment of bladder neurosis, nocturia, pollakiuria accompanied with prostatic hypertrophy or the like, or incontinence accompanied with the same; idiopathic pollakiuria or incontinence accompanied with the same; or urinary frequency or incontinence accompanied with neurogenic bladder dysfunction, unstable bladder, bladder spasm, chronic or acute cystitis, chronic or acute prostatitis or the like. Thus, it is expected to be an effective therapeutic agent for a patient who can not obtain a sufficient efficacy by using a single drug, a patient who desires dose reduction of a drug used for the disease and the like.

### Effect of the Invention

The medicine of the present invention comprising combination of a phenoxyacetic acid derivative represented by the general formula (I) or a pharmaceutically acceptable salt thereof, or a hydrate or solvate thereof and silodosin exerts an excellent effect decreasing the intra-bladder pressure or prolonging the micturition interval. Therefore, the present invention can provide a medicine extremely useful for the prevention or treatment of urinary frequency or incontinence.

### Brief description of Drawings

[Figure 1]

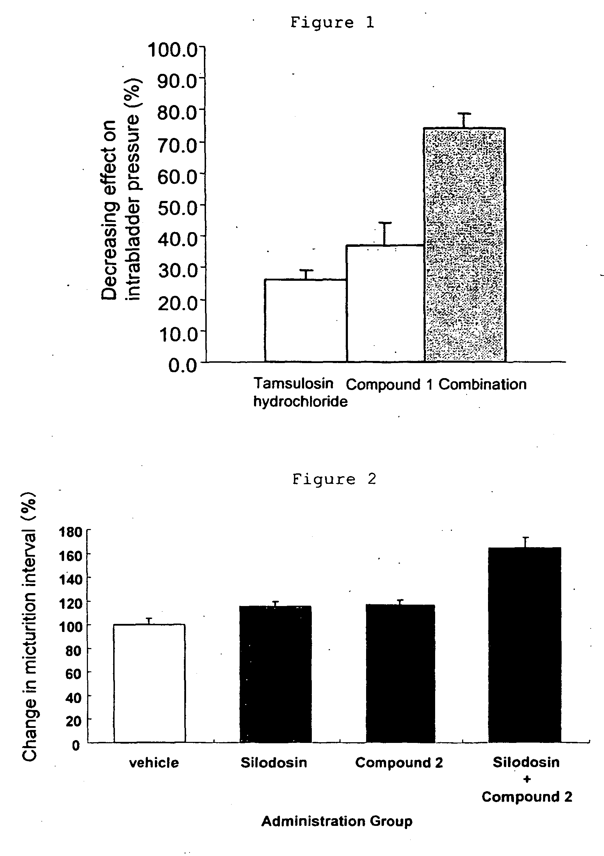

   Figure 1 shows the effect of each drug decreasing the intra-bladder pressure in anesthetized rats. Each column in the figure shows the data of tamsulosin hydrochloride alone, compound 1 alone and a combination of tamsulosin hydrochloride and compound 1, respectively, from the left. The vertical axis indicates the percentage of the effect decreasing the intra-bladder pressure to the maximum decreasing effect by isoproterenol.
[Figure 2]
   Figure 2 shows the effect of each drug prolonging the micturition interval in acetic acid-stimulated rats. Each column in the figure shows the data of vehicle, silodosin alone, compound 2 alone and a combination of silodosin and compound 2, respectively, from the left. The vertical axis indicates the percentage of the effect prolonging the micturition interval to the value before administration.

### Best Mode to practice the Invention

The present invention is further explained in more detail by way of the following Examples, but it is not limited within this content.

### Comparative Example 1

### Intra-bladder pressure management in anesthetized rat

Male rats were anesthetized with urethane. To each rat, tracheal and femoral vein cannulas were inserted. After midline abdominal incision, the ureter on either side was ligated and cut at the proximal end of the ligated portion. After the uretha was ligated, a cannula was inserted into the urinary bladder through the top of the bladder dome. Through a three-way connector, warmed saline was instilled to adjust the intra-bladder pressure to about 10 cmH₂O. The other end of the bladder cannula was connected to a pressure transducer, and intra-bladder pressure was measured. Three mg/kg of midodorin hydrochloride was injected through the femoral vein cannula. Ten minutes after the midodorin hydrochloride injection, tamsulosin hydrochloride (5 µg/kg, iv) or compound 1 (10 µg/kg, iv) was intravenously injected, and the decreasing effect by single administration, compound 1 (10 µg/kg, iv) was intravenously injected to the animal treated with tamsulosin hydrochloride (5 µg/kg, iv) to evaluate the combinational effect. At the last 10 µg/kg of isoproterenol was intravenously injected, and the maximum decreasing effect was set as 100%. As a result, as shown in Figure 1, the effects decreasing the intra-bladder pressure were 26%, 37% and 74% by tamsulosin hydrochloride alone, compound 1 alone and the combination of tamsulosin hydrochloride and compound 1, respectively.

It is found from the results shown in Figure 1 that the combined use of the phenoxyacetatic acid derivative represented by the general formula (I) and the α1-AR blocker exerts a remarkable effect decreasing the intra-bladder pressure by enhancing the effect of the α1-AR blocker by the phenoxyacetic acid derivative represented by the general formula (I) or by enhancing the effect of phenoxyacetic acid derivative by the α1-AR blocker.

### Example 1

### Micturition interval measurement in acetic acid-stimulated rat

Female rats were anesthetized with urethane. After the midline abdominal was incised, and the ureter on either side was ligated and cut, the renal end was kept open. A cannula was inserted into the urinary bladder through the top of the bladder dome and connected to a three-way connecter to establish routes for the intra-bladder pressure measurement and instillation into the bladder. The other end of the bladder cannula was connected to a pressure transducer, and intra-bladder pressure was measured. Saline was continuously instilled into the bladder (3.6 mL/hr). A solution of acetic acid (0.25%) was continuously instilled into the bladder (3.6 mL/hr) to induce shortening of the micturition interval. After stable micturition intervals were obtained, silodosin (0.03 mg/kg), (-)-2-[4-[2-[[(1S, 2R)-2-hydroxy-2-(4-hydroxyphenyl)-1-methylethyl]amino]ethyl]-2,5-dimethylphenoxy] acetic acid (hereinafter referred to as "compound 2") (1 mg/kg) or both of silodosin (0.03 mg/kg) and compound 2 (1 mg/kg) were injected through a femoral venous cannula respectively, and time from when a micturition occurred till when the next micturition was induced (micturition interval) was measured. Mean micturition intervals for 2 times before each drug administration and for all micturition which occurred in 30 minutes after the drug administration were calculated, and the change to the mean value before administration was evaluated. As a result, as shown in Figure 2, the change in the micturition interval were 99.5%, 115.2% and 116.3% in vehicle-treated group (control group), silodosin administration group and compound 2 administration group, respectively, while 163.8% in the combination group.

Two-way layout analysis of veriance was conducted employing the change of the micturition interval as the objective variable and the silodosin administration and compound 2 administration as the factors, and an effect by the combined administration of silodosin and compound 2 was evaluated. As a result, the p value was 0. 0221 in the combined administration of silodosin and compound 2, and a statistically significant interaction was confirmed. Thus, it was demonstrated that the combined administration of silodosin and compound 2 exhibits a synergistic effect prolonging the micturition interval.

From the above results, it is found that combined use of the phenoxyacetatic acid derivative represented by the general formula (I) and the α1-AR blocker exerts a synergistic effect prolonging the micturition interval by enhancing the effect of the α1-AR blocker by the phenoxyacetic acid derivative represented by the general formula (I) or by enhancing the effect of the phenoxyacetic acid derivative by the α1-AR blocker.

### Industrial Applicability

The pharmaceutical compositions used in combination of a phenoxyacetic acid derivative represented by the general formula (I) or a pharmaceutically acceptable salt thereof, or a hydrate or solvate thereof and silodosin exert an excellent effect decreasing the intra-bladder pressure or prolonging the micturition interval. Therefore, the present invention can provide an agent extremely useful for the prevention or treatment of urinary frequency or incontinence.

## Claims

1. A medicine for use in the prevention or treatment of urinary frequency or incontinence, which comprises combination of a phenoxyacetic acid derivative represented by a general formula: 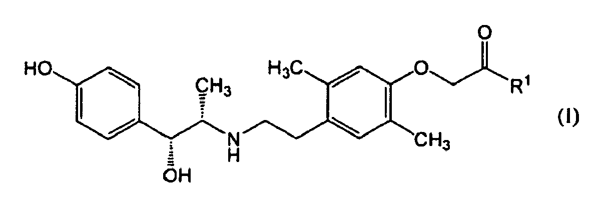 wherein R¹ represents a hydroxy group or a lower alkoxy group, or a pharmaceutically acceptable salt thereof, or a hydrate or solvate thereof and silodosin, wherein the dosage of silodosin or a pharmaceutically acceptable salt thereof is 1 to 16 mg/day as oral dose of silodosin for an adult human.

2. A medicine as claimed in claim 1 wherein the phenoxyacetic acid derivative represented by the general formula (I) is ethyl (-)-2-[4-[2-[[(1S, 2R)-2-hydroxy-2-(4-hydroxyphenyl)-1-methylethyl]amino]ethyl]-2,5-dimethylphenoxy]acetate.

3. A combination formulation for the prevention or treatment of urinary frequency or incontinence, which comprises a phenoxyacetic acid derivative represented by the general formula (I) as defined in claim 1 or a pharmaceutically acceptable salt thereof, or a hydrate or solvate thereof and silodosin, wherein the dosage of silodosin or a pharmaceutically acceptable salt thereof is 1 to 16 mg/day as oral dose of silodosin for an adult human.

4. A combination formulation as claimed in claim 3 wherein the phenoxyacetic acid derivative represented by the general formula (I) is ethyl (-)-2-[4-[2-[[(1S,2R)-2-hydroxy-2-(4-hydroxyphenyl)-1-methylethyl]amino]ethyl]-2,5-dimethylphenoxy]acetate.

## Patentansprüche

1. Medikament zur Verwendung in der Prävention oder Behandlung von Miktionshäufigkeit oder Inkontinenz, welches die Kombination eines durch eine allgemeine Formel: 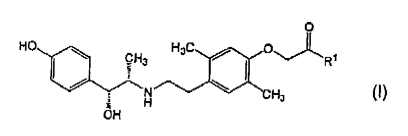 dargestellten Phenoxyessigsäure-Derivates, wobei R¹ eine Hydroxygruppe oder eine Niederalkoxygruppe darstellt, oder eines pharmazeutisch annehmbaren Salzes davon oder eines Hydrates oder Solvates davon, und Silodosin umfasst, wobei die Dosierung von Silodosin oder eines pharmazeutisch annehmbaren Salzes davon 1 bis 16 mg/Tag als orale Dosis an Silodosin für einen erwachsenen Menschen beträgt.

2. Medikament nach Anspruch 1, wobei das Phenoxyessigsäure-Derivat, welches durch die allgemeine Formel (I) dargestellt ist, Ethyl-(-)-2-[4-[2-[[(1S,2R)-2-hydroxy-2-(4-hydroxyphenyl)-1-methylethyl]amino]ethyl]-2,5-dimethylphenoxy]acetat ist.

3. Kombinationsformulierung für die Prävention oder Behandlung von Miktionshäufigkeit oder Inkontinenz, welches ein Phenoxyessigsäure-Derivat, welches durch die in Anspruch 1 definierte allgemeine Formel (I) dargestellt ist, oder ein pharmazeutisch annehmbares Salz davon oder ein Hydrat oder Solvat davon, und Silodosin umfasst, wobei die Dosierung von Silodosin oder eines pharmazeutisch annehmbaren Salzes davon 1 bis 16 mg/Tag als orale Dosis an Silodosin für einen erwachsenen Menschen beträgt.

4. Kombinationsformulierung nach Anspruch 3, wobei das Phenoxyessigsäure-Derivat, weiches durch die allgemeine Formel (I) dargestellt ist, Ethyl-(-)-2-[4-[2-[[(1S,2R)-2-hydroxy-2-(4-hydroxyphenyl)-1-methylethyl]amino]ethyl]-2,5-dimethylphenoxy]acetat ist.

## Revendications

1. Médicament destiné à être utilisé dans la prévention ou le traitement d'une fréquence urinaire ou incontinence, qui comprend une combinaison d'un dérivé de l'acide phénoxyacétique représenté par la formule générale: 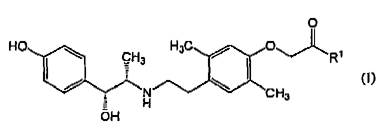 où R¹ représente un groupe hydroxy ou un groupe alcoxy inférieur, ou un sel pharmaceutiquement acceptable de celui-ci, ou un hydrate ou solvate de celui-ci et de la silodosine, où le dosage de silodosine ou d'un sel pharmaceutiquement acceptable de celle-ci est de 1 à 16 mg/jour comme dose orale de silodosine pour un humain adulte.

2. Médicament selon la revendication 1, où le dérivé de l'acide phénoxyacétique représenté par la formule générale (I) est éthyl (-)-2-[4-[2-[[(1S,2R)-2-hydroxy-2-(4-hydroxyphényl)-1-méthyléthyl]amino]éthyl]-2,5-diméthylphénoxy]acétate.

3. Formulation de combinaison pour la prévention ou le traitement d'une fréquence urinaire ou incontinence, qui comprend un dérivé de l'acide phénoxyacétique représenté la formule générale (I) tel que défini dans la revendication 1 ou un sel pharmaceutiquement acceptable de celui-ci, ou un hydrate ou solvate de celui-ci et de la silodosine, où le dosage de silodosine ou d'un sel pharmaceutiquement acceptable de celle-ci est de 1 à 16 mg/jour comme dose orale de la silodosine pour un humain adulte.

4. Formulation de combinaison selon la revendication 3, où le dérivé de l'acide phénoxyacétique représenté par la formule générale (I) est éthyl (-)-2-[4-[2-[[(1S, 2R)-2-hydroxy-y-2-(4-hydroxyphényl)-1-méthyléthyl]amino]éthyl]-2,5-diméthylphoxy]acétate.
